(19) Europäisches Patentamt
European Patent Office
Office européen des brevets

(11) Publication number: 0 069 445
B1

(12) **EUROPEAN PATENT SPECIFICATION**

(45) Date of publication of patent specification: **01.10.86**

(51) Int. Cl.[4]: **C 07 D 235/28**, C 07 D 235/30, C 07 D 235/26, C 07 D 235/24, C 07 D 235/02, C 07 D 233/46, C 07 D 233/42, C 07 D 233/34, C 07 D 233/32, C 07 D 233/30, C 07 D 277/68

(21) Application number: **82302146.4**

(22) Date of filing: **27.04.82**

# (54) Process for making benzimidazoles.

(30) Priority: **01.05.81 US 259732**

(43) Date of publication of application:
**12.01.83 Bulletin 83/02**

(45) Publication of the grant of the patent:
**01.10.86 Bulletin 86/40**

(84) Designated Contracting States:
**BE DE FR GB IT NL**

(56) References cited:
**DE-A-2 214 600**

**CHEMICAL ABSTRACTS, vol.95, no.15, October 12, 1981, page 659, abstract no.132735q, Columbus, Ohio (US) B. ARVENTIEV et al.: "Reaction between o-aminothiophenol and isothiocyanic acid"**

**General and Inorganic Chemistry, Gumnar Hügg, John Wiley p. 600**

**Benzimidazoles and Congeneric Trieyclic compounds, Part 1, J. Wiley, p. 18**

**The file contains technical information submitted after the application was filed and not included in this specification**

(73) Proprietor: **UNIROYAL, INC.**
**1230 Avenue of the Americas Rockefeller Center**
**New York, New York 10020 (US)**

(72) Inventor: **Gencarelli, Richard A.**
**124 Farrel Road Waterbury**
**New Haven Connecticut (US)**
Inventor: **Wheeler, Edward L.**
**126 Clayton Avenue Watertown**
**Litchfield Connecticut (US)**

(74) Representative: **Harrison, Michael Robert et al**
**URQUHART-DYKES & LORD**
**5th Floor Tower House Merrion Way**
**Leeds LS2 8PA West Yorkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a method for making 5-membered ring compounds containing N, O or S atoms by reacting certain (thio)-cyanate salts or cyanamide salts with compounds having $NH_2$, OH or SH functionality vicinal to an $-NH_2$ group, in the presence of an acid except where said cyanate, thiocyanate or cyanamide is an ammonium salt.

The following references are of interest:

1. Org. Syn. Col. Vol. IV, p. 180 which discloses a process for preparing a thiourea from an amine HCl and ammonium thiocyanate.

2. U.S. patent 3,455,948, Stedman, July 15, 1969, particularly the preparation of a thiourea in Example 7.

3. EPO 0 005 276, Nov. 14, 1979, Hoechst AG, describing preparation of a thiourea.

4. Heterocyclic Compounds, Vol. 5, Elderfield, p. 285, disclosing preparation of 2-mercaptobenzimidazoles, etc.

5. Thiourea, D. C. Schroeder, Chem. Rev. Vol. 55, pp. 181—228, which contains a review of thiourea chemistry.

6. EPO 0 012 933, July 9, 1980, Hoechst AG, disclosing production of amino-benzimidazolone.

In one aspect the invention is concerned with a one step process for the preparation of such compounds as 2-hydroxy, 2-amino or 2-mercapto benzimidazoles, benzothiazoles, benzoxazoles, imidazoles, thiazoles or oxazoles by reacting such materials as *o*-phenylenediamines, *o*-aminothiophenols, *o*-aminophenols, 1,2-aminoalcohols or the like with an inorganic cyanate, thiocyanate or cyanamide. An acid is used with the cyanate, thiocyanate or cyanamide in those cases where the anion is not the ammonium ion.

The reaction is run in an organic solvent which enables removal of impurities and color by extracting the product into aqueous sodium hydroxide as its sodium salt and then precipitating the product from the aqueous phase with acid.

In accordance with the present state of the prior art 2-mercaptobenzimidazole is prepared by reacting o-phenylenediamines with carbon disulfide. This reaction generates one mole of highly toxic hydrogen sulfide per mole of product and this hydrogen sulfide must be disposed of. In the process of this invention only a trace of hydrogen sulfide is formed, while the major by-product is ammonia.

The invention may accordingly be characterised as a process for making a benzimidazole having the formula

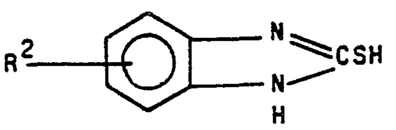 (I)

wherein $R^2$ is hydrogen or methyl characterised in that the process comprises (a) reacting a compound having the formula ZSCN, wherein Z is Na, K or $NH_4$, with a compound having the formula

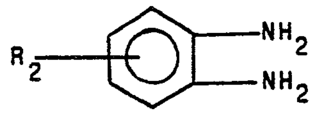

wherein $R^2$ has the meanings above, in the presence of a $C_6$—$C_{10}$ alkanol and, except when Z is $NH_4$, in the presence of an acid, (b) azeotropically removing the water from the reaction mixture, (c) extracting the benzimidazole from the alkanol with aqueous caustic, (d) neutralising the reaction mixture (c) with acid, and (e) isolating the imidazole from the neutralised reaction mixture. The reaction temperature is preferably 90°—200°C.

Preferably the alkanol is hexanol, 2-ethyl-hexanol or decanol and the acid is hydrochloric acid or sulfuric acid. More preferably the alkanol is hexanol or 2-ethylhexanol and the reaction temperature is from 147 to 164°C.

Preferably mixture of compound (1) in aqueous caustic is filtered through charcoal prior to step (b).

The process of the invention is of particular interest as allowing production of such useful compounds as anti-oxidants (e.g., 2-mercaptobenzimidazole) at high yields and purity, at low cost and essentially without hydrogen sulfide by-product.

One preferred practice of the invention may be outlined as follows:

To a suitable reaction vessel equipped with agitation and temperature determining means, as well as with condensing means, are added the solvent, the ammonium or alkali metal thiocyanate and the o-arylenediamine at essentially stoichiometric amounts. If the thiocyanate compound is not an ammonium salt, an about equimolar (to the thiocyanate compound) amount of acid (usually mineral acid such as HCl, $H_2SO_4$, etc.) is charged, and, while agitating, the reaction mixture is heated to about 90—200°C for about 2 to 8 hours. After completion of the reaction, the reaction mixture is treated with aqueous caustic. The aqueous layer is separated and it is neutralized with acid. The precipitate (product) is removed by filtration. In those cases where the product is water soluble, the water is stripped off, and the solid residue is recrystallized from an appropriate solvent such as chloroform.

The concentration of reactants in the solvent is usually within the range of about 200 or less to about 900 or more g/l, preferably about 400 to about 700, more preferably about 600. Reaction temperatures are ordinarily within the range of about 110° to about 200°C or more, depending on the particular reactants, preferably about 140° to about 180°C, more preferably from about 140° to about 160°. Reaction times within the range of from about 2 hours or less to about 12 hours or more, preferably about 4 to about 8 hours, more preferably about 6 hours. The reaction may be carried out at any desired pressure, usually atmospheric, although sub-atmospheric or superatmospheric pressure (e.g. 10 atmospheres or more) may be used if desired.

Of particular interest is a process including the following product purification steps:

(a) Extraction of product from the organic phase with aqueous caustic;

(b) Neutralizing the caustic solution from (a) with acid;

(c) Isolating the product from step (b) by filtration.

Preferably, prior to step (b), the mixture of product in hot aqueous caustic is treated with charcoal in order to remove impurities.

The following examples will serve to illustrate the practice of the invention in more detail.

Example 1

Preparation of 2-Mercapto-4(5)-methylbenzimidazole

To a one liter round-bottom, three-necked flask equipped with an agitator, a thermometer, a reflux condenser and a Stark and Dean trap were added 122 g (1.0 mole) ortho-toluenediamine (mixture of 34% 2,3-toluenediamine, 63% 3,4-toluenediamine, 2% other isomers and 1% water), 244 g 2-ethylhexanol and 145.2 g 50% aqueous ammonium thiocyanate. The mixture was heated to reflux (ca. 164°C), and the water was azeotroped out for about one hour. The reaction mixture was kept at 164°C for 6 hours. The mixture was cooled to about 100°C, and 41 g of solid sodium hydroxide in 500 ml of water was added while stirring. The mixture was transferred to a separating funnel, and the aqueous layer was removed. The alcohol layer was washed with 500 ml of water, and the water drawn off. The combined water layers were neutralized with acid and the title product was removed by filtration and dried.

Example 2

Preparation of 2-Mercapto-4(5)-methylbenzimidazole

To a two-liter, three-necked round bottom flask equipped with a thermometer, agitator, dropping funnel and a Stark and Dean trap with condenser were added 122 g (1.0 mol) o-toluenediamine, 81 g (1.0 mol) sodium thiocyanate and 500 ml n-hexanol. While stirring, 84 ml concentrated hydrochloric acid was added, and the mixture was then heated to 160°C. The water present was azeotroped out of the flask during said heating period (ca. 1 hour). The mixture was refluxed (ca. 160°C) for five hours and then no further heat was applied. 170 ml of 6N sodium hydroxide (diluted to 250 ml) was slowly added to the reaction mixture which continued to reflux. After cooling the aqueous layer was removed and neutralized. A small amount (ca. 8 g) of a white solid was isolated by filtration. The hexanol portion was treated with about two liters of water. This aqueous portion was acidified causing a white solid product to precipitate. The product was isolated by filtration and dried. Total product yield was 159.5 g (97.3% of theory).

Examples 3—8

Following essentially the procedures of Examples 1 and 2 respectively, additional preparations were undertaken using various reactants, solvents and process conditions, all summarized in Table I. In examples 3—7 the product is 2-mercapto-4(5)-methylbenzimidazole. In Example 8 the product is 2-mercaptobenzimidazole.

The results demonstrate the usefulness of the process of this invention for the preparation of 2-mercaptobenzimidazoles in an easy and efficient manner.

TABLE I

| Example | 3 | 4 | 5 | 6 | 7 | 8 |
|---|---|---|---|---|---|---|
| TDA(1), mol | 1 | 1 | 1 | 1 | 1 | — |
| PDA(2), mol | — | — | — | — | — | 1 |
| ZSCN, mole | 1 | 1 | 1 | 1 | 1 | 1 |
| Z of ZSCN | Na | Na | $NH_4$ | $NH_4$ | $NH_4$ | $NH_4$ |
| Acid: HCl, mol | — | 1 | — | — | — | — |
| Acid: $H_2SO_4$, mol | 1 | — | — | — | — | — |
| Solvent | hexanol | hexanol | 2EH(3) | hexanol | decanol | 2EH |
| Solvent, ml | 150 | 220 | 222 | 220 | 170 | 220 |
| Reaction temp., °C | 147 | 162 | 164 | 160 | 164 | 164 |
| Reaction time, hrs. | 19 | 5.5 | 6 | 6 | 6 | 5 |
| Yield, g | 150 | 162 | 152 | 152 | 130 | 128 |
| Yield, %(4) | 92 | 99 | 93 | 93 | 79 | 85 |

Remarks:

(1) o-toluenediamine
(2) o-phenylenediamine
(3) 2-ethylhexanol
(4) of theoretical yield based on appropriate (1) or (2)

**Claims**

1. A process for making a benzimidazole having the formula

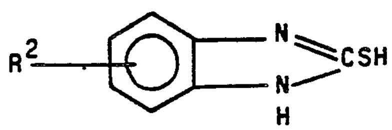

(I)

wherein $R^2$ is hydrogen or methyl characterised in that the process comprises (a) reacting a compound having the formula ZSCN, wherein Z is Na, K or $NH_4$, with a compound having the formula

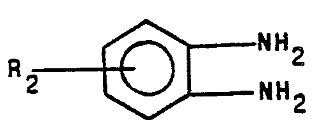

wherein $R^2$ has the meanings above, in the presence of a $C_6$—$C_{10}$ alkanol and, except when Z is $NH_4$, in the presence of an acid, (b) azeotropically removing the water from the reaction mixture, (c) extracting the benzimidazole from the alkanol with aqueous caustic, (d) neutralising the reaction mixture (c) with acid, and (e) isolating the imidazole from the neutralised reaction mixture.

2. A process according to claim 1 characterised in that the reaction temperature is from 90° to 200°C.

3. A process according to claim 1 or claim 2 characterised in that said alkanol is hexanol, 2-ethyl-hexanol or decanol and said acid is hydrochloric acid or sulfuric acid.

4. A process according to any of the preceding claims characterised in that said alkanol is hexanol or 2-ethylhexanol and the reaction temperature is from 147 to 164°C.

5. A process according to any of the preceding claims characterised in that prior to step (b) the material of (I) in aqueous caustic is filtered through charcoal.

**Patentansprüche**

1. Verfahren zur Herstellung eines Benzimidazols, das die Formel

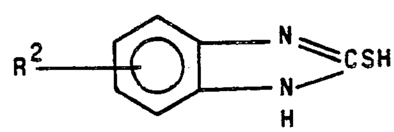

(I)

hat, worin $R^2$ Wasserstoff oder Methyl ist, dadurch gekennzeichnet, daß bei dem Verfahren (a) eine Verbindung, die die Formel ZSCN hat, worin Z Na, K oder $NH_4$ ist, in Gegenwart eines $C_6$- bis $C_{10}$-Alkanols und — mit Ausnahme des Falls, daß Z $NH_4$ ist — in Gegenwart einer Säure mit einer Verbindung, die die Formel

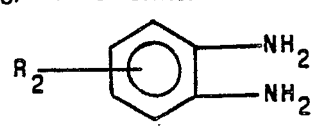

hat, worin $R^2$ die vorstehend angegebenen Bedeutungen hat, umgesetzt wird, (b) das Wasser

aus der Reaktionsmischung azeotropisch entfernt wird, (c) das Benzimidazol mit wäßrigem Ätzalkali aus dem Alkanol extrahiert wird, (d) die Reaktionsmischung (c) mit Säure neutralisiert wird und (e) das Imidazol aus der neutralisierten Reaktionsmischung isoliert wird.

2. Verfahren nach Anspruch 1, dadurch gekennzeichnet, daß die Reaktionstemperatur 90 bis 200°C beträgt.

3. Verfahren nach Anspruch 1 oder Anspruch 2, dadurch gekennzeichnet, daß das erwähnte Alkanol Hexanol, 2-Ethylhexanol oder Decanol ist und daß die erwähnte Säure Salzsäure oder Schwefelsäure ist.

4. Verfahren nach einem der vorhergehenden Ansprüche, dadurch gekennzeichnet, daß das erwähnte Alkanol Hexanol oder 2-Ethylhexanol ist und daß die Reaktionstemperatur 147 bis 164°C beträgt.

5. Verfahren nach einem der vorhergehenden Ansprüche, dadurch gekennzeichnet, daß vor Schritt (b) die Mischung von (I) in wäßrigem Ätzalkali durch künstliche Kohle bzw. Holzkohle filtriert wird.

**Revendications**

1. Procédé de préparation d'un benzimidazole ayant pour formule

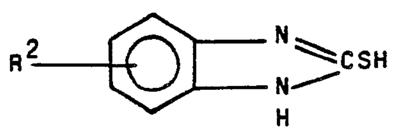

(I)

où $R^2$ est hydrogène ou méthyle, caractérisé en ce que le procédé comprend (a) la réaction d'un composé ayant la formule ZSCN, où Z est Na, K ou $NH_4$, avec un composé ayant pour formule

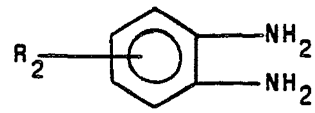

où $R^2$ a les significations ci-dessus, en présence d'un alcanol $C_6$—$C_{10}$ et, à l'exception du cas où Z est $NH_4$, en présence d'un acide, (b) l'enlèvement azéotropique de l'eau du mélange réactionnel, (c) l'extraction du benzimidazole de l'alcanol par un produit caustique aqueux, (d) la neutralisation du mélange réactionnel (c) avec un acide et (e) l'isolement de l'imidazole du mélange réactionnel neutralisé.

2. Procédé selon la revendication 1, caractérisé en ce que la température de la réaction est de 90° à 200°C.

3. Procédé selon la revendication 1 ou la revendication 2, caractérisé en ce que ledit alcanol est l'hexanol, le 2-éthyl-hexanol ou le décanol et ledit acide est l'acide chlorhydrique ou l'acide sulfurique.

4. Procédé selon l'une quelconque des revendications précédentes, caractérisé en ce que ledit alcanol est l'hexanol ou le 2-éthylhexanol et la température de la réaction est comprise entre 147 et 164°C.

5. Procédé selon l'une quelconque des revendications précédentes, caractérisé en ce qu'avant l'étape (b), le mélange de (I) dans le produit caustique aqueux est filtré à travers du charbon de bois.